(19) Europäisches Patentamt
European Patent Office
Office européen des brevets

(11) Veröffentlichungsnummer: **0 009 777**
B1

(12) # EUROPÄISCHE PATENTSCHRIFT

(45) Veröffentlichungstag der Patentschrift:
**05.05.82**

(51) Int. Cl.[3]: **C 07 C 175/00,** A 61 K 31/165

(21) Anmeldenummer: **79103684.1**

(22) Anmeldetag: **28.09.79**

(54) **Retinsäure- und 7,8-Dehydro-retinsäure-N-(carboxy)-phenyl-amide, Verfahren zu ihrer Herstellung und diese enthaltende pharmazeutische Mittel.**

(30) Priorität: **07.10.78 DE 2843811**

(43) Veröffentlichungstag der Anmeldung:
**16.04.80 Patentblatt 80/8**

(45) Bekanntmachung des Hinweises auf die Patenterteilung:
**05.05.82 Patentblatt 82/18**

(84) Benannte Vertragsstaaten:
**AT BE CH DE FR GB IT LU NL SE**

(56) Entgegenhaltungen:

**FR-A-2 212 135**
**FR-A-2 293 193**

(73) Patentinhaber: **BASF Aktiengesellschaft,**
***Carl-Bosch-Strasse 38, D-6700 Ludwigshafen (DE)***

(72) Erfinder: **Paust, Joachim, Dr., Ringstrasse 3,**
**D-6701 Neuhofen (DE)**
Erfinder: **Nuerrenbach, Axel, Dr., Koenigsberger**
**Strasse 7, D-6718 Gruenstadt (DE)**

**EP 0 009 777 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Retinsäure- und 7,8-Dehydro-retinsäure-N-(carboxy)-phenyl-amide, Verfahren zu ihrer Herstellung und diese enthaltende pharmazeutische Mittel

Die Erfindung betrifft all-E- und 13-Z-Retinsäure-Verbindungen der Formel I und II, in denen

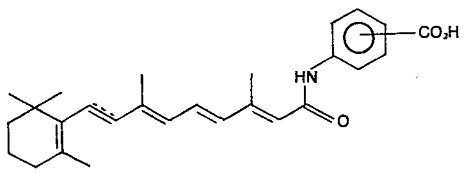

I

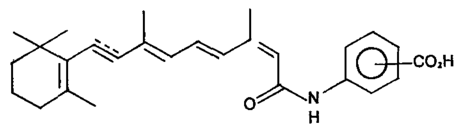

II

die gestrichelte Linie eine chemische Bindung oder zwei Wasserstoffatome darstellt und die Carboxylgruppe am aromatischen Ring in ortho-, meta- oder p-Position stehen kann, die Herstellung dieser Verbindungen, diese Verbindungen enthaltende pharmazeutische Zubereitungen und ihre Verwendung als Arzneimittel.

Aus der DE-OS 2 102 586 ist bekannt, dass Retinsäureamide, die am Amidstickstoff beispielsweise eine Benzyl- oder Phenylgruppe als Substituenten aufweisen, pharmakologische Wirkung zeigen. Für diese Verbindungen wird beschrieben, dass sie zur topischen und systemischen Therapie von Praekanzerosen und Karzinomen sowie zur topischen und systemischen Prophylaxe von Karzinomen verwendet werden können. Sie können auch für die Therapie von Akne, Psoriasis und anderen mit verstärkter oder pathologisch veränderter Verhornung einhergehenden dermatologischen Affektionen sowie bei Ekzemen und bei Affektionen der Schleimhäute verwendet werden. Als Einzelverbindungen werden in der DE-OS 2 102 586 u.a. beispielsweise Retinsäure-N-ethylamid, Retinsäure-N-benzyl--amid und Retinsäure-N-phenylamid beschrieben.

Aus der DE-OS 2 300 107 gehen Retinsäureamide insbesondere mit substituierten Phenylresten für etwa den gleichen Verwendungszweck hervor. Als Einzelverbindungen werden beispielsweise Retinsäure-(p-ethoxy)-anilid und Retinsäure-N-(p-ethoxycarbonyl)-phenylamid beschrieben.

Nachteilig an diesen bekannten Retinsäureamiden ist ihre geringe therapeutische Breite. Die Substanzen zeigen entweder eine vergleichsweise geringe Aktivität, oder sie wirken schon in vergleichsweise geringen Dosen toxisch. So kann beispielsweise Retinsäure-N--ethylamid schon in $2 \times 10^{-9}$ molaren Konzentrationen die durch Vitamin A-Hypovitaminose induzierte Keratinisierung von Hamster-Tracheen-Gewebe wieder rückgängig machen. Die Methodik hierzu kann G. H. Clamon et al Nature 250, 64-66 (1974) oder M. B. Sporn et al Nature 253, 47-50 (1975) entnommen werden. Die Keratinisierung wird als praekanzerotischer Prozess angesehen. Das Retinsäure-N-ethylamid wirkt bereits in einer Konzentration von $9 \times 10^{-7}$ molar toxisch auf Tracheenknorpelgewebe vom Hamster in Kultur, wie von M. B. Sporn et al in Nature 263, 110-113 (1976) beschrieben ist. Retinsäure-N-(p-ethoxycarbonyl)-phenylamid andererseits zeigt geringe celluläre Toxizität, vermag jedoch die Keratinisierung von Hamster-Tracheen-Gewebe erst in $10^{-8}$ molarer Konzentration zu heilen.

Aus der FR-A-2 293 193 gehen Desmethylvitamin-A-Säurederivate, Verbindungen, bei denen in β-Stellung zum Carbonyl der Säuregruppe die bei Retinsäure übliche Methylgruppe fehlt, hervor. Auch diese Verbindungen befriedigen in ihrer Wirkung nicht immer.

Es wurde nun gefunden, dass die all-E- und 13-Z-Retinsäure-N-(carboxy)-phenylamide der Formel I und II trotz verhältnismässig geringer struktureller Unterschiede besonders wertvolle pharmakologische Eigenschaften aufweisen, da sie einen wesentlich grösseren therapeutischen Index besitzen.

Die tumorhemmende Wirkung der erfindungsgemässen Verbindungen ist signifikant. Man beobachtet eine Wachstumskontrolle bei in vitro kultivierten Zellen, die beispielsweise nach der Methodik, wie sie von R. Lotan et al. im Journal of the National Cancer Institute 60 (1978) Seiten 1035 - 1041 beschrieben wird, nachgewiesen werden kann. Die erfindungsgemässen Verbindungen inhibieren die Proliferation von spontan chemisch oder durch Viren transformierter Zellen in Gewebekultur, vorzugsweise verwendet man die S 91 Melanoma Zellinie.

Beispielsweise weist das all-E-Retinsäure-N--(p-carboxy)-phenylamid eine ebenso geringe Toxizität wie der entsprechende Ethylester auf. Bei der Prophylaxe von Praekanzerosen an keratinisiertem Hamster-Tracheen-Gewebe ist die freie Säure jedoch noch in $10^{-10}$ molarer Konzentration wirksam und damit wesentlich aktiver als der Ethylester.

Entsprechende Resultate wurden erhalten mit
all-E-Retinsäure-N-(m-carboxy)-phenylamid
all-E-Retinsäure-N-(o-carboxy)-phenylamid
all-E-7,8-Dehydro-retinsäure-N-(p-carboxy)--phenylamid
all-E-7,8-Dehydro-retinsäure-N-(m-carboxy)--phenylamid
all-E-7,8-Dehydro-retinsäure-N-(o-carboxy)--phenylamid
13-Z-Retinsäure-N-(p-carboxy)-phenylamid
13-Z-Retinsäure-N-(m-carboxy)-phenylamid
13-Z-Retinsäure-N-(o-carboxy)-phenylamid.

Von den erfindungsgemässen Verbindungen sind all-E-Retinsäure-N-(p-carboxy)-phenylamid, all-E-7,8-Dehydro-retinsäure-N-(p-carboxy)-phenylamid und 13-Z-Retinsäure-N-(p-carboxy)-phenylamid und insbesondere all-E-Retinsäure-N--(o-carboxy)-phenylamid und 13-Z-Retinsäure-N--(o-carboxy)-phenylamid bevorzugt.

Die erfindungsgemässen Verbindungen werden in an sich üblicher Weise hergestellt durch Umsetzung eines funktionellen Säurederivates der all-E- oder 13-Z-Retinsäure oder der all-E- oder 13-Z-7,8-Dehydro-retinsäure mit ortho-, meta- oder para-Aminobenzoesäure oder eines ihrer Säurederivate, das anschliessend in üblicher Weise zur Säure hydrolysiert wird.

Als funktionelle Derivate der all-E-oder 13-Z--Retinsäure oder der all-E- oder 13-Z-7,8-Dehydro-retinsäure werden insbesondere die Ester oder Säurehalogenide, vorzugsweise die Säurechloride, verwendet. Als Derivate der ortho-, meta- oder para-Aminobenzoesäure lassen sich besonders die entsprechenden Ester, z.B. Ethylester, einsetzen. Die bei der Umsetzung der Retinsäurechloride mit den Aminobenzoesäureestern zunächst entstehenden Retinsäure-N-(alkoxycarbonyl)-phenylamide können durch alkalikatalysierte Hydrolyse der Estergruppen in üblicher Weise in die Phenylcarbonsäure überführt werden.

Die Herstellung der Retinsäurechloride und ihre weitere Umsetzung mit einer Aminobenzoesäure oder einer ihrer Ester wird vorzugsweise in einem inerten organischen Lösungsmittel, wie einem Dialkylether oder einem chlorierten aliphatischen Kohlenwasserstoff, beispielsweise Diethylether oder Methylenchlorid, oder in Gemischen dieser Lösungsmittel und in Gegenwart von Basen bei Temperaturen zwischen −25 und 25°C durchgeführt.

Bevorzugt arbeitet man in Diethylether oder in Gemischen und verwendet die ein- bis zweifache molare Menge Pyridin als Base. Zweckmässigerweise arbeitet man unter Ausschluss von Sauerstoff und Feuchtigkeit, z.B. unter Stickstoff als Inertgas.

Die Aufarbeitung des Endprodukts erfolgt in an sich üblicher Weise. Eine bevorzugte Reinigung besteht jedoch in der Umkristallisation.

Die erfindungsgemässen Verbindungen können aufgrund der genannten pharmakologischen Eigenschaften bei der topischen und systemischen Therapie und Prophylaxe von Praekanzerosen und Karzinomen der Haut, Schleimhäute und innerer Organe sowie bei der topischen und systemischen Therapie von Akne, Psoriasis und anderen mit pathologisch veränderter Verhornung einhergehenden dermatologischen Erkrankungen verwendet werden.

Ein bevorzugtes Indikationsgebiet ist die prophylaktische und therapeutische Behandlung von Praekonzerosen und Tumoren der Blase, der Brustdrüse, der Haut und der Schleimhäute.

Dementsprechend sind ein weiterer Gegenstand der Erfindung pharmazeutische Mittel, die eine Verbindung der Formel I oder II neben üblichen pharmazeutischen Trägerstoffen oder Verdünnungsmitteln und gegebenenfalls üblichen pharmazeutisch-technischen Hilfsstoffen als Wirkstoff enthalten, und die Verwendung einer Verbindung der Formel I oder II zur Herstellung eines Arzneimittels.

Die Herstellung der therapeutischen Mittel oder Zubereitungen mit üblichen Trägerstoffen oder Verdünnungsmitteln und gegebenenfalls üblichen pharmazeutisch-technischen Hilfsstoffen, entsprechend der gewünschten Applikationsart mit einer zur Anwendung geeigneten Dosierung erfolgt in an sich üblicher Weise, insbesondere durch Vermischen.

Die therapeutischen Mittel enthalten die erfindungsgemäss zu verwendenden Verbindungen bei lokaler Anwendung in 0,001 bis 1,0%iger Konzentration, bevorzugt in 0,01 bis 0,1%iger Konzentration, und bei systemischer Anwendung vorzugsweise in einer Einzeldosis von 0,1 bis 5 mg. Dabei kommen als Tagesdosis 5 bis 100 mg in Betracht, wobei die Dosierungen je nach Art und Schwere der Erkrankung, der Zubereitung und der Applikation variieren können.

Es werden die üblichen galenischen Zubereitungen verwendet, für die orale Applikation beispielsweise Tabletten, Filmtabletten, Dragees, Kapseln, Pillen, Pulver, Granulate, Lösungen oder Suspensionen. Für die äusserliche Anwendung kommen insbesondere Pasten, Salben, Gele, Cremes, Lotionen, Puder, Lösungen oder Emulsionen und Sprays in Betracht.

Die erfindungsgemässen Mittel gelangen insbesondere zur innerlichen und äusserlichen Anwendung. Sie werden vorzugsweise oral oder lokal appliziert.

Üblicherweise verwendete pharmazeutisch technische Hilfsstoffe sind beispielsweise für die lokale Anwendung Alkohole, wie Isopropanol, oxäthyliertes Ricinusöl oder oxäthyliertes hydriertes Ricinusöl, Polyacrylsäure, Glycerinmonostearat, Paraffinöl, Vaseline, Wollfett, Polyäthylenglykol 400, Polyäthylenglykol 400-Stearat sowie äthoxylierter Fettalkohol, für die systemische Anwendung Saccharose, Milchzucker, Propylenglykol und Äthanol, Stärke, Talkum, Polyvinylpyrrolidon.

Weitere übliche Zusätze sind beispielsweise Konservierungsmittel, Antioxydantien, geschmackverbessernde Zusätze, Stabilisierungsmittel, Emulgiermittel, Gleitmittel, Netzmittel usw. Voraussetzung ist, dass alle bei der Herstellung pharmazeutischer Zubereitungen verwendeten Stoffe untoxisch und mit den verwendeten Wirkstoffen verträglich sind (vgl. L. G. Goodman, A. Gilman, The Pharmacological Basis of Therapeutics).

Beispiel 1

all-E-Retinsäure-N-(p-carboxy)-phenylamid:

75 Gewichtsteile Retinsäure werden in 1000 Raumteilen Diethylether suspendiert, mit 21,9 Gewichtsteilen Pyridin versetzt und bei 0°C tropfenweise mit einer Lösung von 33 Gewichtsteilen Thionylchlorid in 150 Raumteilen Diethylether versetzt. Man rührt 2 Stunden bei 0°C weiter, trennt vom kristallinen Pyridiniumhydrochlorid durch Filtrieren ab und lässt das Filtrat so zu einer Suspension von 34 Raumteilen p-Aminobenzoesäure in 41,8 Gewichtsteilen Pyridin, 250 Raumteilen Diethylether und 250 Raumteilen Methylenchlorid fliessen, dass die Reaktionstemperatur $-20°C$ nicht überschreitet. Anschliessend wird 2 Stunden bei ca 25°C nachgerührt.

Zur Aufarbeitung wäscht man das Reaktionsgemisch mit 250 Raumteilen eiskalter 1 normaler Salzsäure und mit 250 Raumteilen gesättigter Kochsalzlösung, arbeitet wie üblich auf und kristallisiert den Rückstand aus 1200 Raumteilen Aceton um. Nach der Trocknung (50°C, 0,1 Torr, 5 Stunden) erhält man 75 Gewichtsteile all-E-Retinsäure-N-(p-carboxy)-phenylamid, Fp. 199 bis 201°C.

Beispiele 2 bis 9

Nach der in Beispiel 1 angegebenen Vorschrift erhält man in vergleichbaren Ausbeuten:
all-E-Retinsäure-N-(m-carboxy)-phenylamid
Fp. 193 bis 194°C aus Aceton
all-E-Retinsäure-N-(o-carboxy)-phenylamid
Fp. 172 bis 173°C aus Acetonitril
13-Z-Retinsäure-N-(p-carboxy)-phenylamid
Fp. 165 bis 166°C aus Essigester
13-Z-Retinsäure-N-(m-carboxy)-phenylamid
Fp. 152 bis 154°C aus Essigester
13-Z-Retinsäure-N-(o-carboxy)-phenylamid
Fp. 144 bis 146°C aus Essigester
all-E-7,8-Dehydro-retinsäure-N-(p-carboxy)-phenylamid
Fp. 235 bis 237°C aus Acetonitril-Methanol
all-E-7,8-Dehydro-retinsäure-N-(m-carboxy)-phenylamid
Fp. 211 bis 213°C aus Acetonitril-Methanol
all-E-7,8-Dehydro-retinsäure-N-(o-carboxy)-phenylamid
Fp. 202 bis 205°C aus Acetonitril-Methanol.

Die (Isomeren-)Reinheit der hergestellten Verbindungen lässt sich HPLC-analytisch bestimmen und liegt in der Regel > 96%. Die Strukturzuordnung basiert auf der H-NMR-Spektroskopie.

Geeignete pharmazeutische Zubereitungen oder Arzneistoffträger für die äusserliche Anwendung sind z.B.:

Beispiel 10

Lösung

| | |
|---|---|
| all-E-Retinsäure-N-(p-carboxy)-phenylamid | 0,25 g |
| oxäthyliertes hydriertes Ricinusöl (in Deutschland im Handel erhältlich als ®Cremophor RH 40, Hersteller BASF AG, Ludwigshafen) | 35,0 g |
| Polyäthylenglykol 400 | 35,0 g |
| oxäthyliertes Ricinusöl (in Deutschland im Handel erhältlich als ®Softigen 767, Hersteller Chemische Werke, Witten) | 10,0 g |
| demineralisiertes Wasser | ad 100,0 g |

Cremophor RH 40 und Softigen 767 werden gemischt und auf 70°C erhitzt. Die Wirksubstanz wird unter Rühren gelöst und Polyäthylenglykol 400 zugesetzt. Die Lösung wird dann auf 40°C abgekühlt und unter Rühren wird langsam auf 40°C erhitztes Wasser zugesetzt. Die fertige Lösung wird filtriert und in z.B. 100 ml Flaschen gefüllt.

Beispiel 11

Creme

| | |
|---|---|
| all-E-7,8-Dehydro-retinsäure-N-(p-carboxy)-phenylamid | 0,1 g |
| Butylhydroxytoluol | 0,1 g |
| Glycerinmonostearat | 11,0 g |
| Polyäthylenglykol 400-Stearat | 6,0 g |
| äthoxylierter Fettalkohol | 4,0 g |
| Paraffinöl | 10,0 g |
| p-Hydroxybenzoesäurester (in Deutschland im Handel erhältlich als ®Nipasteril, Hersteller Nipalaboratorium Hamburg) | 0,2 g |
| Parfumöl | 0,1 g |
| demineralisiertes Wasser | ad 100,0 g |

Die Fette werden geschmolzen und die feinstgepulverte Wirksubstanz sowie Butylhydroxytoluol unter Rühren bei 65°C darin verteilt (Lösung I). Das Wasser wird mit dem Nipaester aufgekocht und auf 65°C abgekühlt (Lösung II). In kleinen Anteilen wird Lösung II unter gutem Rühren in Lösung I einemulgiert. Nach dem Abkühlen auf 45°C wird das Parfumöl zugesetzt und die Emulsion unter Rühren auf Zimmertemperatur abgekühlt. Die fertige Creme wird in innenschutzlackierte Tuben abgefüllt.

Beispiel 12

Gel

| | |
|---|---|
| 13-Z-Retinsäure-N-(p-carboxy)-phenylamid | 0,01 g |
| Butylhydroxytoluol | 0,1 g |
| oxäthyliertes Ricinusöl | 35,0 g |
| Isopropanol | 20,0 g |
| Polyacrylsäure (in Deutschland im Handel erhältlich als ®Carbopol, Hersteller Goodrich, Hamburg) | 1,5 g |
| Triäthanolamin | 0,002 g |
| p-Hydroxybenzoesäureester | 0,2 g |
| demineralisiertes Wasser | ad 100,0 g |

Das Cremophor EL wird auf 60°C erhitzt, der Wirkstoff und das Butylhydroxytoluol unter Rühren gelöst und das Isopropanol, in dem die Nipaester gelöst wurden, zugemischt (Lösung I). Carbopol wird unter kräftigem Rühren in dem Wasser verteilt (Lösung II). Lösung II wird unter gutem Rühren in kleinen Anteilen zu Lösung I gemischt. Der pH-Wert des Gemisches wird mit Triäthanolamin auf 4,5 eingestellt. Das fertige Gel wird in innenschutzlackierte Tuben abgefüllt.

Für die systemische Anwendung besonders geeignete Zubereitungen oder Arzneistoffträger sind z.B.:

Beispiel 13

Tropfen

| | |
|---|---|
| all-E-Retinsäure-N-(p-carboxy)-phenylamid | 0,1 g |
| Propylenglykol | 25,0 g |
| Äthylalkohol | ad 50,0 g |

Äthylalkohol und Propylenglykol werden mit-

einander gemischt und die Wirksubstanz unter Erwärmen auf 35°C und unter Rühren gelöst. Nach Filtration wird die Lösung in dunkle Tropfflaschen abgefüllt.

Beispiel 14

Hartgelatinekapseln

| | |
|---|---|
| 13-Z-Retinsäure-N-(p-carboxy)-phenylamid | 1 mg |
| Milchzucker | ad 0,25 g |

Die Bestandteile werden gesiebt, gemischt und auf einer geeigneten Kapselfüll- und -verschlussmaschine in Hartgelatinekapseln der Grösse 2 gefüllt.

**Patentansprüche für die Vertragsstaaten:**
BE, CH, DE, FR, GB, IT, LU, NL, SE

1. Retinsäure- und 7,8-Dehydro-retinsäure-N--(carboxy)-phenylamide der Formel I und II

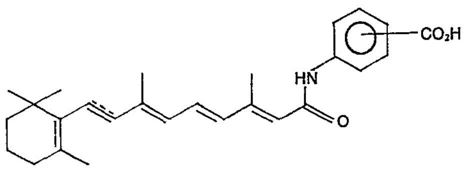

I

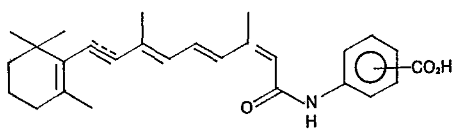

II

in denen die gestrichelte Linie eine chemische Bindung oder zwei Wasserstoffe darstellt und die Carboxylgruppe am aromatischen Ring in o-, m- oder p-Position steht.

2. all-E-Retinsäure-N-(o-carboxy)-phenylamid.

3. 13-Z-Retinsäure-N-(o-carboxy)-phenylamid.

4. Verfahren zur Herstellung von Verbindungen der Formel I und II nach Anspruch 1 - 3, dadurch gekennzeichnet, dass man ein funktionelles Säurederivat der all-E- oder 13-Z-Retinsäure oder der all-E- oder 13-Z-7,8-Dehydro-retinsäure mit o-, m- oder p-Aminobenzoesäure oder eines ihrer Säurederivate in an sich üblicher Weise umsetzt und gegebenenfalls das erhaltene Säurederivat in üblicher Weise zur Säure hydrolysiert.

5. Verfahren nach Anspruch 4 zur Herstellung von all-E-Retinsäure-N-(o-carboxy)-phenylamid.

6. Verfahren nach Anspruch 4 zur Herstellung von 13-Z-Retinsäure-N-(o-carboxy)-phenylamid.

7. Pharmazeutisches Mittel, gekennzeichnet durch einen Gehalt an einer Verbindung der Formel I oder II nach Anspruch 1 neben üblichen Trägerstoffen oder Verdünnungsmitteln.

8. Pharmazeutisches Mittel, gekennzeichnet durch einen Gehalt an all-E-Retinsäure-N-(o--carboxy)-phenylamid nach Anspruch 7 neben üblichen Trägerstoffen und Verdünnungsmitteln.

9. Pharmazeutisches Mittel, gekennzeichnet durch einen Gehalt an 13-Z-Retinsäure-N-(o--carboxy)-phenylamid nach Anspruch 7 neben üblichen Trägerstoffen und Verdünnungsmitteln.

10. Verbindung der Formel I oder II nach Anspruch 1 als Arzneimittel bei der topischen und systemischen Therapie und Prophylaxe von Praekonzerosen und Karzinomen der Haut, Schleimhäute und innerer Organe und bei dermotologischen Erkrankungen.

**Patentanspruch für den Vertragsstaat:** AT

Verfahren zur Herstellung von Retinsäure- und 7,8-Dehydro-retinsäure-N-(carboxy)-phenylamiden der Formel I und II

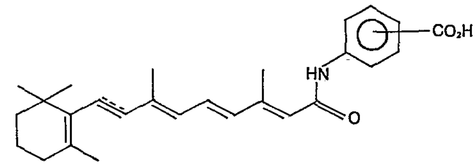

I

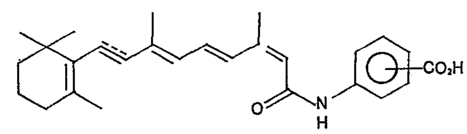

II

in denen die gestrichelte Linie eine chemische Bindung oder zwei Wasserstoffe darstellt und die Carboxylgruppe am aromatischen Ring in o-, m- oder p-Position steht, dadurch gekennzeichnet, dass man ein funktionelles Säurederivat der all-E- oder 13-Z-Retinsäure oder der all-E oder 13-Z-7,8-Dehydro-retinsäure mit o-, m- oder p-Aminobenzoesäure oder eines ihrer Säurederivate in an sich üblicher Weise umsetzt und gegebenenfalls das erhaltene Säurederivat in üblicher Weise zur Säure hydrolysiert.

**Claims for the Contracting States:**
BE, CH, DE, FR, GB, IT, LU, NL, SE

1. Retinic acid N-(carboxy)-phenylamides and 7,8-dehydro-retinic acid N-(carboxy)-phenylamides of the formulae I and II

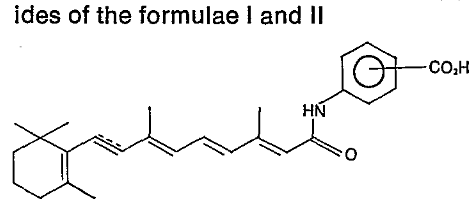

I

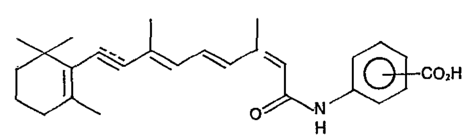

II

where the broken line is a chemical bond or two hydrogen atoms, and the carboxyl group in the aromatic ring is in the ortho-, meta- or para-position.

2. all-E-Retinic acid N-(o-carboxy)-phenylamide.

3. 13-Z-Retinic acid N-(o-carboxy)-phenylamide.

4. A process for the preparation of a compound of the formula I or II as claimed in claims 1 to 3, characterized in that a functional acid derivative of all-E- or 13-Z-retinic acid or of all-E- or 13-Z-7,8-dehydro-retinic acid is reacted with o-, m- or p-aminobenzoic acid or an acid derivative thereof in the conventional manner, and, where appropriate, the resulting acid derivative is hydrolyzed to the acid in the conventional manner.

5. A process as claimed in claim 4 for the preparation of all-E-retinic acid N-(o-carboxy)-phenylamide.

6. A process as claimed in claim 4 for the preparation of 13-Z-retinic acid N-(o-carboxy)-phenylamide.

7. A pharmaceutical formulation characterized by a content of a compound of the formula I or II as claimed in claim 1, in addition to conventional carriers or diluents.

8. A pharmaceutical formulation characterized by a content of all-E-retinic acid N-(o-carboxy)-phenylamide as claimed in claim 7, in addition to conventional carriers and diluents.

9. A pharmaceutical formulation characterized by a content of 13-Z-retinic acid N-(o-carboxy)-phenylamide as claimed in claim 7, in addition to conventional carriers and diluents.

10. A compound of the formula I or II as claimed in claim 1 as a drug in the topical and systemic therapy and prophylaxis of pre-cancerous conditions and carcinomas of the skin, mucous membranes and internal organs, and in dermatological disorders.

**Claim for the Contracting State:** AT

A process for the preparation of retinic acid N-(carboxy)-phenylamides and 7,8-dehydro-retinic acid N-(carboxy)-phenylamides of the formulae I and II

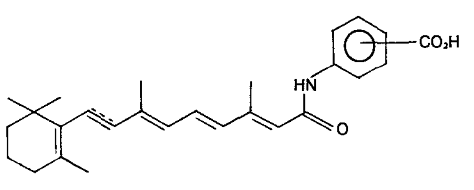

I

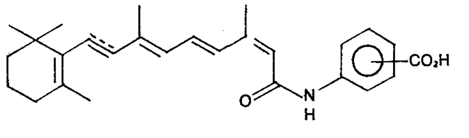

II

where the broken line is a chemical bond or two hydrogen atoms, and the carboxyl group in the aromatic ring is in the ortho-, meta- or para-position, characterized in that a functional acid derivative of all-E- or 13-Z-retinic acid or all-E- or 13-Z-7,8-dehydro-retinic acid is reacted with o-, m- or p-aminobenzoic acid or an acid derivative thereof in the conventional manner, and, where appropriate, the resulting acid derivative is hydrolyzed to the acid in the conventional manner.

**Revendications pour les Etats contractants:** BE, CH, DE, FR, GB, IT, LU, NL, SE

1. N-(carboxy)-phénylamide d'acide rétinoïque et de 7,8-déhydrorétinoïque de formules I et II

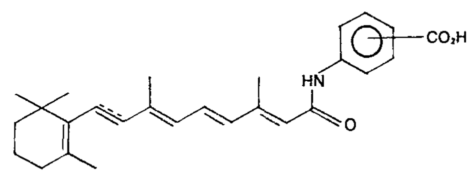

I

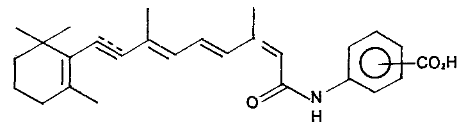

II

dans lesquelles la ligne pointillée représente une liaison chimique ou deux hydrogènes, et le groupe carboxyle se trouve en position o-, m- ou p- sur le noyau aromatique.

2. N-(o-carboxy)-phénylamide d'acide all-E-rétinoïque.

3. N-(o-carboxy)-phénylamide d'acide 13-Z-rétinoïque.

4. Procedé de préparation de composés de formules I et II, selon les revendications 1 - 3, caractérisé par le fait qu'on fait réagir, de manière connue en soi, un dérivé d'acide fonctionnel de l'acide all-E- ou 13-Z-rétinoïque ou de l'acide all-E- ou 13-Z-7,8-déhydrorétinoïque avec de l'acide o-, m- ou p-aminobenzoïque ou l'un de ses dérivés d'acide, et on transforme éventuellement le dérivé d'acide obtenu en acide par hydrolyse, de manière usuelle.

5. Procédé selon la revendication 4 pour la préparation de N-(o-carboxy)-phénylamide d'acide all-E-rétinoïque.

6. Procédé selon la revendication 4 pour la préparation de N-(o-carboxy)-phénylamide d'acide 13-Z-rétinoïque.

7. Agent pharmaceutique, caractérisé par le fait qu'il contient un composé de formules I ou II selon la revendication 1 à côté de véhicules ou diluants usuels.

8. Agent pharmaceutique, caractérisé par le fait qu'il contient un N-(o-carboxy)-phénylamide d'acide all-E-rétinoïque selon la revendication 7 à côte de véhicules et diluants usuels.

9. Agent pharmaceutique, caractérisé par le fait qu'il contient un N-(o-carboxy)-phénylamide d'acide 13-Z-rétinoïque selon la revendication 7 à côte de véhicules et diluants usuels.

10. Composé de formules I ou II selon la revendication 1, en tant que médicament pour la thérapeutique topique ou systémique et la prophylaxie de précancéroses et carcinomes de la peau, des muqueuses et des organes internes et pour les maladies dermatologiques.

**Revendication pour l'Etat contractant:** AT

Procédé de préparation de N-(carboxy)-phénylamide d'acide rétinoïque et de 7,8-déhydrorétinoïque de formules I et II

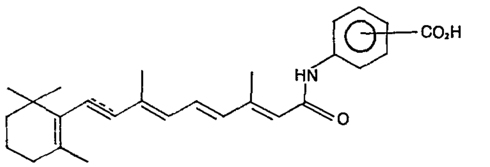

I

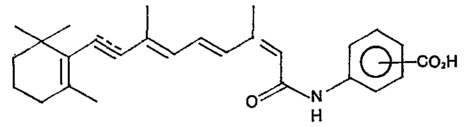

II

dans lesquelles la ligne pointillée représente une liaison chimique ou deux hydrogènes, et le groupe carboxyle se trouve en position o-, m- ou p- sur le noyau aromatique, caractérisé par le fait que l'on fait réagir, de manière connue en soi, un dérivé d'acide fonctionnel de l'acide all-E- ou 13-Z-rétinoïque ou de l'acide all-E- ou 13-Z-7,8-déhydrorétinoïque avec de l'acide o-, m- ou p-aminobenzoïque ou l'un de ses dérivés d'acide, et on transforme éventuellement le dérivé d'acide obtenu en acide par hydrolyse, de manière usuelle.